# EUROPEAN PATENT APPLICATION

(11) **EP 0 805 346 A1**
(43) Date of publication of application: **05.11.1997**
(21) Application number: 96107021.6
(22) Date of filing: 03.05.1996
(51) Int. Cl.: G01N 3/20, G01N 33/34

(54) **Device for measuring bending strength of sheet material**

(71) Applicant: Yoshizawa Industry Inc., Nagaoka-shi, Niigata 940 (JP)
(72) Inventor: Yoshizawa, Akinori, c/o Yoshizawa Industry Inc., Nagaoka-shi, Niigata 940 (JP)
(74) Representative: Strehl Schübel-Hopf Groening & Partner

(57) **Abstract**

A device for measuring bending strength of a sheet material (D) in a predetermined direction is disclosed. The device has a clamp (12), a load sensor (20), a revolving unit (18), a data-processing unit, and a data-displaying unit (24). The clamp (12) has a tip that extends in a direction perpendicular to the predetermined direction and holds one end portion of the sheet material (D). The load sensor (20) is positioned relatively below the other end portion of the sheet material being held by the clamp (12). The revolving unit (18) for revolving the clamp (12) is provided on one end in the direction along the tip of the clamp (12). The data-processing unit for storing the resistance force to be detected by the load sensor (20) by contacting with the sheet material (D) during a period of revolving the clamp (12) and information concerning the position of the clamp (12) traveled by the revolving unit (18), by which stored data is processed and outputted as output data in a predetermined form. Furthermore, the data-displaying unit (24) displays the output data.

## Description

### BACKGROUND OF THE INVENTION

### FIELD OF THE INVENTION

The present invention relates to a device for measuring bending strength of sheet material, such as corrugated fiberboard or paperboard, at a crease (score) portion or a blank portion thereof.

### DESCRIPTION OF THE PRIOR ART

For manufacturing corrugated fiberboard containers, in general, a base board for corrugated fiberboard is processed into a continuous band-shaped corrugated fiberboard by passing it through a corrugator. The obtained sheet is cut into a plurality of corrugated fiberboard sheets having a predetermined length and then stocked to keep for further processing. After that, each of the stocked sheets is subjected to a printing process and also cut into a desired shape by means of a die board which also makes the slits and creases to be required built into the container. Then each die cut sheet (die cut box) is formed into a designated corrugated fiberboard container and stocked in a folded state. In case of need, furthermore, the folded corrugated fiberboard container is extended and built into an assembled container in the shape of a box. Hereinafter, the assembled corrugated fiberboard container will be referred as a corrugated box.

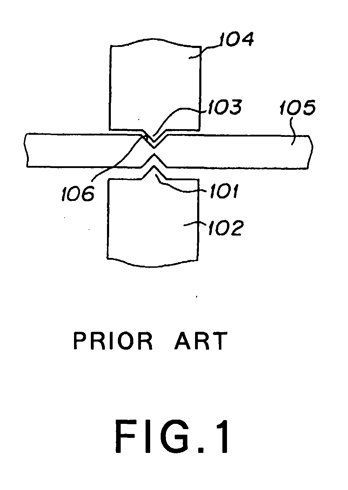

Referring now to Fig. 1, there is shown a device for the purpose of explaining one of the examples of the conventional methods for making creases in corrugated fiberboard. In this case, the creased portions can be provided as lines for folding flaps of the corrugated fiberboard container to make a corrugated box. According to the conventional method, as shown in the figure, A crease 106 can be shaped by constant pressing through a combination of an anvil 102 having a protrusion 101 and an extrusion die 104 having a edge portion 103 corresponding to that protrusion 101. In the case of making a crease by using a flexoprinter folder gluer (FFG) or the like, an anvil having a flat surface without any protrusion is used. Recently, furthermore, it has been known that a crease can be prepared as a series of holes bored through one liner and corrugating media of corrugated fiberboard, the remaining liner stays intact, by means of a laser beam with a pulse oscillation mode.

Regarding the aforementioned crease of the corrugated fiberboard, the warping force of the creased portion varies among the methods to be selected for making the crease, for example at the time of bending the flap. In addition, the warping force of the creased portion also varies among the materials of the corrugated fiberboard and with the conditions of the formed crease, for example the warping force of the creased portion varies with a large or small amount of clearance between the edge portion 103 and the protrusion 101 (or the anvil's surface if it is not provided thereon) when the crease is shaped by forcing with the extrusion die 104 having the edge portion 103. When the crease is prepared as a series of holes by means of the laser beam as described above, the warping force of the creased portion varies with the differences in size of each hole or in spacing between adjacent holes.

Consequently, the following problems can be occur in accordance with the aforementioned variations in the warping force of the creased portion.

In a case where the corrugated boxes are used for packing a wide variety of commodity products or the like on packing lines under automatic control, the step of fixing the top and bottom flaps of each corrugated box together by an adhesive tape is performed as a last step in the packing process. Thus the height of the empty corrugated box depends on the warping force of the creased portion, the box's own weight, the symmetry or proportion of the box's shape or dimensions, and so on. When the warping force of the creased portion is too big, each flap of the corrugated box tends to extend higher than the acceptable height or width of the box with respect to the packing line. As the corrugated box is too high, it is difficult to place the empty corrugated box correctly on the line and to keep the box on the line without bumping into something, resulting in the above packing line being stopped. Recently, in particular, automatic high-speed packaging apparatuses have been introduced increasingly in the fields of the food and drink industries and the like, so that wrap around cases made of paperboard or corrugated fiberboard to be used in those fields suffer serious damages arising out of qualities of the creases formed thereon. Therefore the amount of money for the damage may increased enormously. Accordingly, manufacturers of paper containers and corrugated fiberboard have been poured all their energies into a quality-control method with a particular emphasis on the strength of the crease.

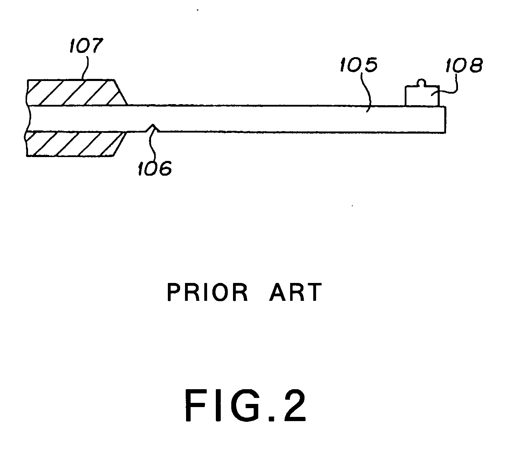

A conventionally well-known method for measuring the bending strength of creases will be described hereinafter with reference to Fig. 2. The method comprises, as shown in Fig. 2, the steps of holding one end of a corrugated fiberboard 105 having a crease 106 by a holder 107, loading a weight on the other end of the corrugated fiberboard 105, and making an assessment of the strength of the crease 106 at the time of folding by means of the heaviness of the weight 108. Therefore, the above method is able to estimate yield strength of the creased portion but not to estimate warping force thereof.

Strength of any portions of the corrugated fiberboard except the creased one is directly related to the strength of the corrugated box, so that it is very important to understand the bending strength of the corrugated fiberboard easily from the point of quality control. It is noted that this point is also important for various kinds of sheet materials including paperboard, plastic sheet, film, metal thin plate, honey-comb sheet, and so on.

### SUMMARY OF THE INVENTION

An object of the present invention is to provide a device providing for easily measuring the bending strength of corrugated fiberboards, various kinds of sheet materials including paperboard and the like, or creased portions thereof.

Another object of the present invention is to provide a device having excellent portability for measuring the bending strength of sheet material in any location at any time.

There is provided a device for measuring bending strength of a sheet material in a predetermined direction, comprising:
a clamp having a tip that extends in a direction perpendicular to said predetermined direction and holds one end portion of said sheet material;
a load sensor which is positioned relatively below another end portion of said sheet material being held by said clamp;
a revolving means for revolving said clamp, which is provided on one end in a direction along said tip of said clamp;
a data-processing means for storing the resistance force to be detected by said load sensor by contacting with said sheet material during a period of revolving said clamp and information concerning the position of said clamp traveled by said revolving means, by which stored data is processed and outputted as output data in a predetermined form; and
a data-displaying means for displaying said output data.

Here, said revolving means may be a handle for manually revolving said clamp.

The revolving means may be capable of revolving said clamp in a predetermined angle between over 90° to nearly 180°.

The data-displaying means may be removably connected with a connector for connection and disconnection to a main body.

The said connector is capable of being connected with a personal computer.

The said data-displaying means is a printer.

The said sheet material is a corrugated fiberboard.

The said sheet material is a paperboard.

By using the device of the present invention, bending strength of corrugated fiberboard, paperboard, or creased portion thereof can be easily measured in any location at any time, so that the measuring device of the present invention extremely contributes to the control qualities of these sheet materials and also to facilitate sales thereof and so on.

The above and other objects, effects, features and advantages of the present invention will become more apparent from the following description of embodiments thereof taken in conjunction with the accompanying drawings.

### BRIEF DESCRIPTION OF THE DRAWINGS

Fig. 1 is a schematic sectional view of the device for carrying out the process of making a crease on the corrugated fiberboard;
Fig. 2 is a schematic sectional view of the device for carrying out the conventional way of making an assessment of the creased portion of the corrugated fiberboard;
Fig. 3 is a plane view of the device for measuring the bending strength of sheet materials for illustrating a preferred embodiment of the present invention;
Fig. 4 is a front view of the device for measuring the bending strength of sheet materials for illustrating one of the preferred embodiment of the present invention;
Fig. 5 is a partial cutaway view of the device shown in Fig. 4;
Fig. 6 is a graphical representation with a table of the results obtained by the measurement performed by the device of the preferred embodiment;
Fig. 7 is a graphical representation of the results obtained by the measurement performed by the device of the preferred embodiment;
Fig. 8 is a graphical representation of the results obtained by the measurement performed by the device of the preferred embodiment;
Fig. 9 is a plane view of the device for measuring bending strength of sheet materials for illustrating another preferred embodiment of the present invention;
Fig. 10 is a graphical representation of the results obtained by the measurement performed by the device of another preferred embodiment;
Fig. 11 is a graphical representation of the results obtained by the measurement performed by the device of another preferred embodiment;
Fig. 12 is a graphical representation of the results obtained by the measurement performed by the device of another preferred embodiment; and
Fig. 13 is a graphical representation of the results obtained by the measurement performed by the device of another preferred embodiment.

### DETAILED DESCRIPTION OF PREFERRED EMBODIMENTS

The present invention will be described in detail with respect to preferred embodiments, and it will now be that changes and modifications may be made without departing from the invention in its broader aspects, and it is the invention, therefore, in the appended claims to cover all such changes and modifications as fall within the true spirit of the invention.

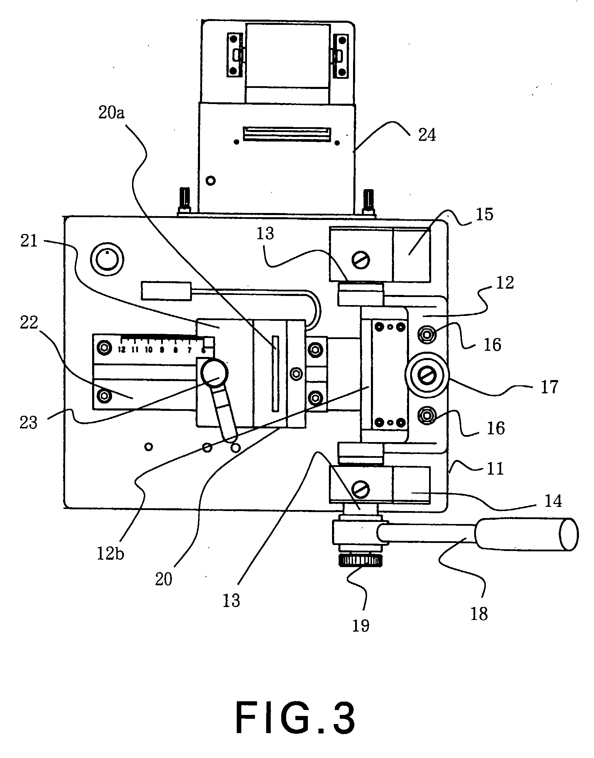

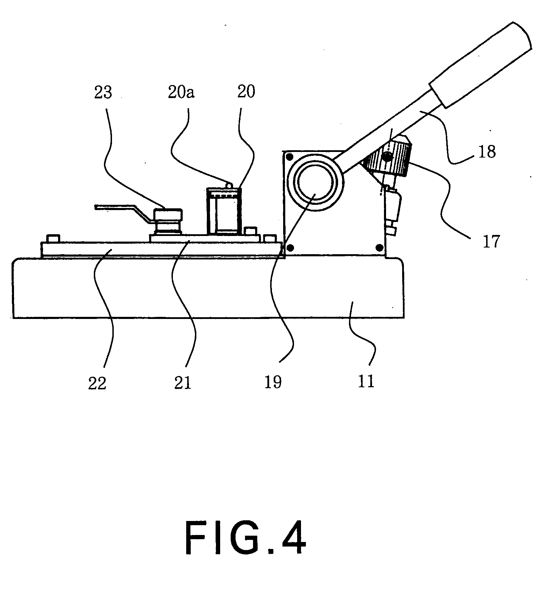

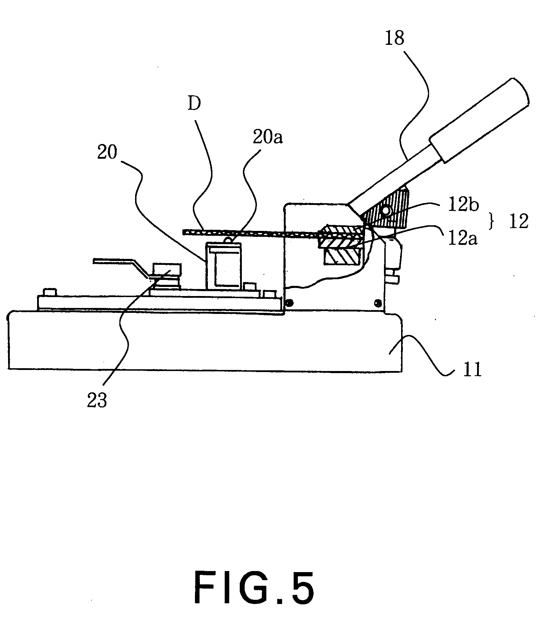

Figs. 3 to 5 illustrate a device for measuring bending strength of corrugated fiberboard as one of the preferred embodiments of the present invention, in which Fig. 3 is a plane view, Fig. 4 is a front view, and Fig. 5 is a partial sectional front view of the device. The device shown in these figures is designed as compact and lightweight so as to be easily carried or moved to any place at any time.

As shown in the figures, the device comprises a base 11 in which a data-processing unit (not shown) is imbedded. The data-processing unit is provided for processing the results obtained by measurement to print them as characteristic values including a yielding value (gf), a yielding angle (deg), an initial gradient (gf/deg), and a graph representing the relationship between a revolution angle (deg) and a resistance value (gf). On one end portion of the base 11, there is provided a clamp 12 for clamping one end of the corrugated fiberboard D as a test sheet. The clamp 12 is rotatably connected to a shaft 13 as the center of the rotation. In addition, the shaft 13 is rotatably supported by bearing pads 14, 15 which are fixed on both-sides of the clamp 12, respectively. The clamp 12 comprises a fixed click 12a being secured to the shaft 13 and a movable click 12b that performs closing and opening with respect to the fixed click 12a. The movable click 12b is supported by a pair of pins 16 and a clamp-adjusting screw 17 so as to be movable only in the direction of opening and closing. Thus the movable click 12 be can be opened or closed by revolving the clamp-adjusting screw 17. The clamp 12 of the present embodiment is in the type of a ratchet, so that the force exerted on the movable click 12b toward the fixed click 12a does not exceed a predetermined level. According to such construction, therefore, the corrugated fiberboard D held by the clamp 12 of the present embodiment does not become flat.

As shown in the figures, furthermore, one end portion of the shaft 13 is protruded from the bearing pad 14 to outside the device and equipped with a handle 18 for revolving the clamp 12. The handle 18 can be fixed to an end portion of the shaft 13 in the direction of revolution by means of a handle-fixing screw 19. Nearly in the center of the base 11, there is provided a load sensor 20 having a load-sensing portion 20a. As shown in the figures, the load sensor 20 protrudes upward from the surface of the base 11, while the load-sensing portion 20a extends in the direction corresponding to the width of the test sheet. Furthermore the load sensor 20 is secured on a mobile platform 21 which is able to shift its position along a guide rail 22 toward the right and left sides of the figure when viewed from the front thereof. After the movement, the mobile platform 21 can be fixed at a predetermined position by mean of a fixing screw 23.

For measuring bending strength of the corrugated fiberboard by using the device described above, a test sheet of the corrugated fiberboard, with a size of 50 mm in width and the measuring length plus 40 mm or more in length, is prepared as a sample. Then one end thereof is held by the clamp 12. In this condition, the handle 18 is in the position shown in Figs. 4 and 5, while the free end portion of the test sheet D having the end portion held by the clamp 12 is in the position part from the load sensing portion 20a of the load sensor 20 so as to be spaced therefrom (see Fig. 5). Then the handle 18 is revolved in a predetermined angle between over 90° to nearly 180° in a counterclockwise direction. As the handle 18 starts to revolve, the other end of the test sheet D touches to the load sensing portion 20a of the load sensor 20. The measurement can be completed by further revolving the handle in the predetermined angle from that position. A force f (a resistance value) applied on the load sensor 20 is measured so as to correspond to the revolving angle.

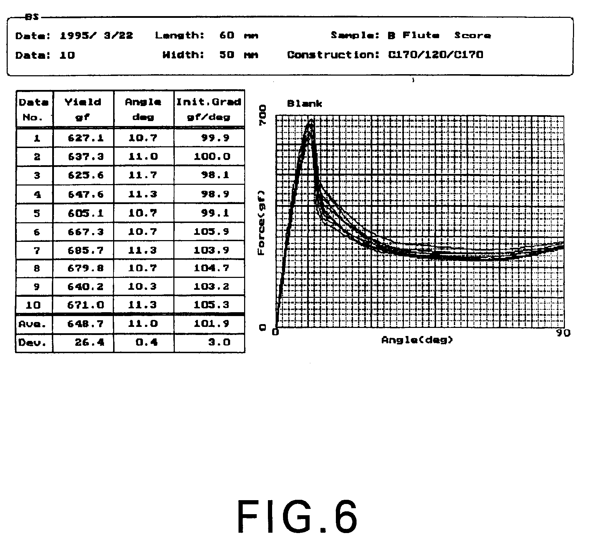

According to the present embodiment, as shown in the figures, there is provided a printer 24 on the opposite side of the base 11 with respect to the handle 18 equipped thereon. The printer 24 is able to print out the results processed by the aforementioned data-processing unit in accordance with the data obtained by the measurement. The results may be printed out as, for example in the form of a table showing the characteristic values or of a graph showing the relation between the revolution angle (deg) and the resistance value (gf). According to the present embodiment, furthermore, the printer 24 is detachable and attachable for easily taking on the road. The connector for the printer 24 is in the type of RS-232C for general purpose use, so that it can be connected to a personal computer or the like for the purpose of not only easily displaying at-a-glance charts of the characteristics values and the characteristic graphs as shown in Figs. 6 to 12 but also editing, comparing, and storing the data, for easily carrying out an advanced analysis thereof and so on.

For the sake of attaining the objectives of the present invention, in spite of with or without the printer, the device must be equipped with means for recognizing the data in any location at any time if required. Such means is not restricted to the printer but also for example a data-displaying device such as a liquid crystal display may be used.

Accordingly, we performed the measurements for estimating bending-strength of the corrugated fiberboard by using the measuring device of the present embodiment. In addition, we inputted a plurality of obtained results of several samples into a personal computer for data-processing to make an assessment of bending strength. For further consideration, several examples of the outputs from the personal computer are shown in Figs. 5 to 8.

The data shown in Fig. 6 is of the measurement for bending strength of a blank portion (not the creased portion) of the test sheet (5.0 cm in width), in which ten samples are used. In this measurement, furthermore, the measuring length of the blank portion under the measurement is 6.0 cm which corresponds to the distance between the tip of the click portion 12a and 12b of the clamp 12 and the load sensing portion 20a of the load sensor 20. In this embodiment, the blank portion is of a score of the corrugated fiberboard (class B), wherein the score is in the direction perpendicular to a flute.

The obtained results of the measurement are shown in the table including a yielding value (gf), a yielding angle (deg), and an initial gradient (gf/deg) and in a graph representing the relationship between a revolution angle (deg) and a resistance value (gf).

Each of the characteristic values and its definition will be explained as follows.

### Yielding value (gf)

The yielding value represents a load at the time of bending the sheet material by revolving the handle from the position where the test sheet is horizontal or nearly horizontal after placing the test sheet in the measuring device. In addition, the yielding value is dependent on the stiffness of the material, so that a higher the stiffness of the sheet material, the likelier it becomes that the higher yielding value has been attained. As for the corrugated fiberboard, the yielding value may vary by the effect of bonding conditions (i.e., bonding requirements, amount of applied paste, or the like) in spite of using the same flute and the same material.

### Yielding angle (deg)

The yielding angle represents an angle corresponding to the yielding value. In the process of bending the blank sheet, the higher the stiffness of the sheet material, the likelier it becomes that the lower yielding angle has been attained.

### Initial gradient (gf/deg)

The initial gradient represents variations in the resistance just after starting the measurement (i.e., at the time that the revolution angle is almost at zero). It is readily calculated from the gradient of the tangent to the leading edge of the data by processing the data obtained within a period between the instant when the measurement is started and the instant when yielding angle is attained. Therefore, the initial gradient can be obtained with respect to the bending of the corrugated fiberboard, paperboard, or the like, so that it stands for capableness of being bent with ease or it stands for difficulty to bend (i.e., bending strength).

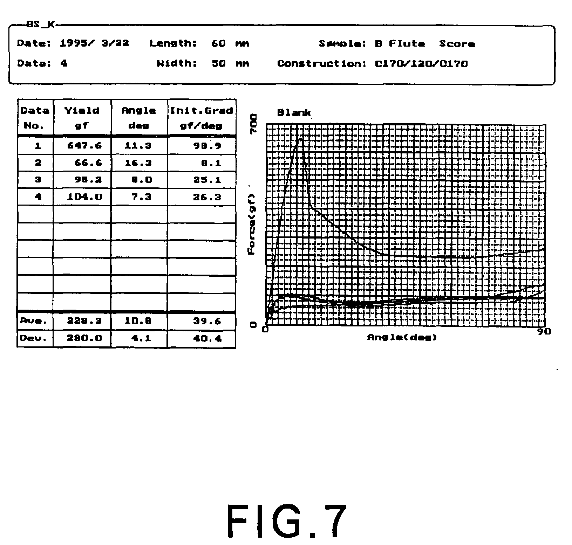

The data shown in Fig. 7 corresponds to the results of the measurement for making an assessment of bending strength of the creased portion of each test sheet (5.0 cm in width and 6.0 cm in measuring length), in which the data No. 1 is of the blank portion and the data Nos. 2 to 4 are of the creased portions of the corrugated fiberboard (class B).

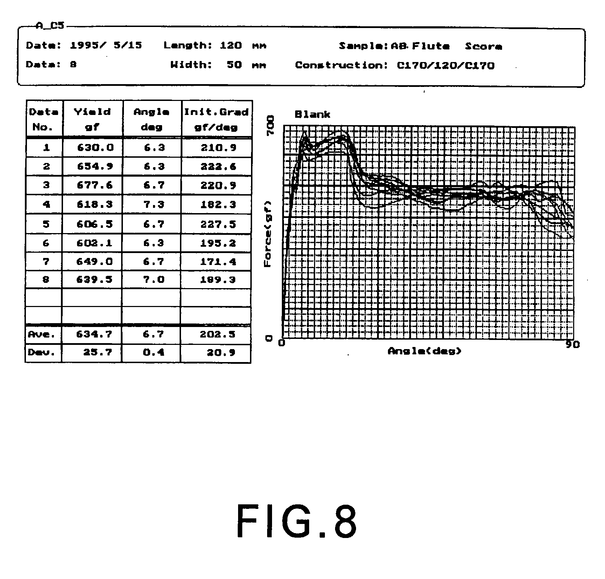

The data shown in Fig. 8 corresponds to the results of the measurement for making an assessment of bending strength of the score of each test sheet (5.0 cm in width and 12.0 cm in measuring length) prepared from the corrugated fiberboard (class B). In this measurement, eight samples are used.

In the measuring device of the present embodiment, the handle 18 is manually revolved. However, the results of the measurement are not affected by the bending rate when the speed of revolution is in the range of 50 degree/second or less. Just as in the case of the aforementioned embodiment, it is possible to make an assessment of yielding strength of the creased portion, warping force of the folded creased portion, mechanical characteristics of the creased portion, and so on.

By accumulating the data in an appropriate storage means, furthermore, a box strength can be estimated by using the yielding value of the bending of the blank portion. Thus the measuring device of the present invention will be able to support the design of the packaging with corrugated fiberboard.

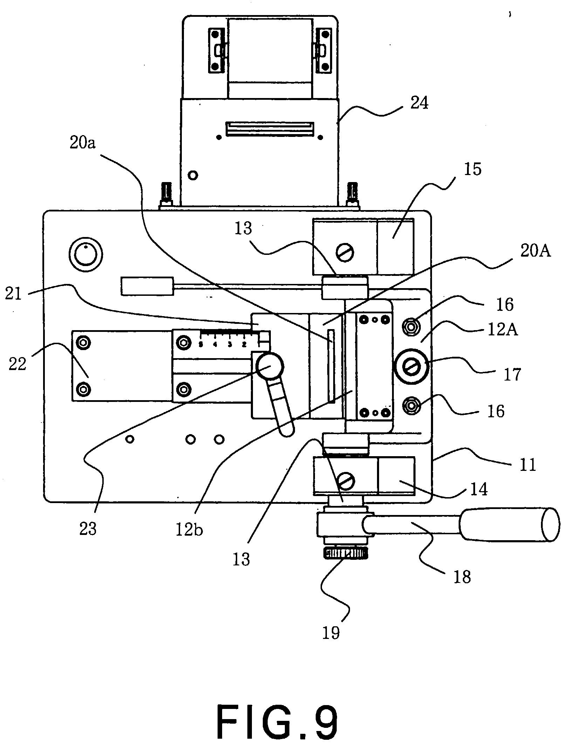

For measuring bending strength of the paperboard, Fig. 9 shows a bending-strength measuring device as another preferred embodiment of the present invention. The measuring device is constructed in the same way as that of the aforementioned embodiment with the exception that follows. In the present embodiment, that is, an allowable load of the load sensor 20A is maintained within narrow limits and also the load sensor 20A is positioned at a point closer to the clamp 12A, compared with that of the first preferred embodiment. In addition, the clamp 12A of the present embodiment is not a ratchet type but a normal clamp.

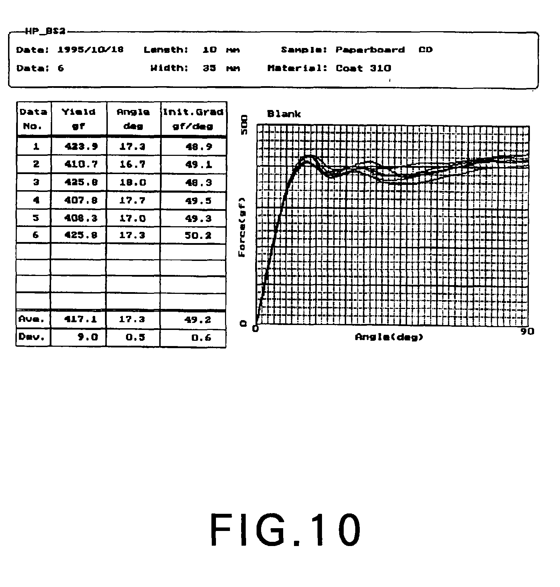

Referring now to Figs. 10 to 13, there are represented the results of the measurement of the bending strength of the paperboard by using the measuring device of the present embodiment.

In these figures, Fig. 10 shows the results of the measurement under the condition that a test sheet (2.5 cm in width) of the paperboard is bent in a vertical position with respect to cross-grain. The results are obtained from eight samples with the measuring length of 1.5 cm.

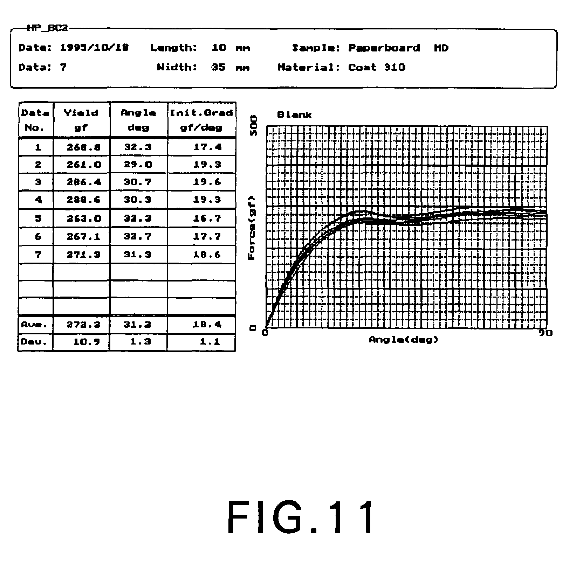

Fig. 11 shows the results of the measurement under the condition that a test sheet of 2.5 cm in width is bent in a parallel position with respect to cross-grain. The results are obtained from nine samples with the measuring length of 1.5 cm.

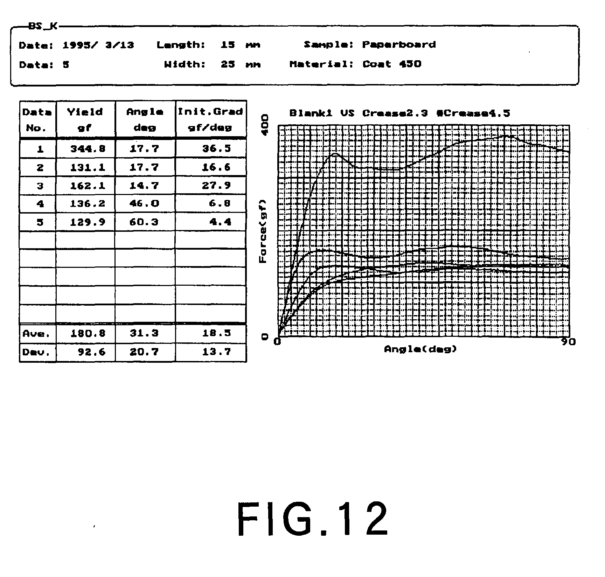

Fig. 12 shows the results of the measurement under the same condition as that of Fig. 10 except that the results are obtained by measuring bending strength of the blank portion (data No. 1), creased portion (data Nos. 2 and 3), and perforated portion (data Nos. 4 and 5) of the paperboard.

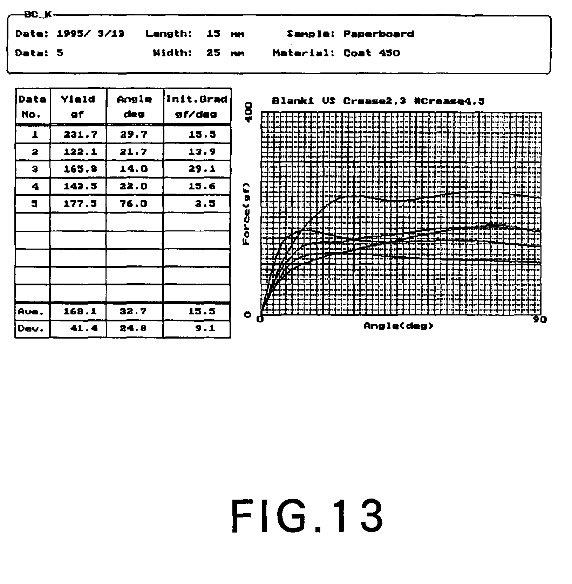

Furthermore, Fig. 13 shows the results of the measurement under the same condition as that of Fig. 11 except that the results are obtained by measuring bending strength of the blank portion (data No. 1), creased portion (data Nos. 2 and 3), and perforated portion (data Nos. 4 and 5) of the paperboard.

## Claims

1. A device for measuring the bending strength of sheet material in a predetermined direction, characterised by
clamping means (12) having a tip that extends in a direction perpendicular to said predetermined direction and holds one end portion of said sheet material (D),
a load sensor (20) positioned below another end portion of said sheet material (D),
means (18) for revolving said clamping means (12) the revolving means being provided on one end in a direction along said tip of said clamping means,
data-processing means for storing the resistance force to be detected by said load sensor (20) by contacting with said sheet material (D) during a period of revolving said clamping means (12), and information on the position of said clamping means moved by said revolving means (18), the stored data being processed and output as output data in a predetermined form, and
means (24) for displaying said output data.

2. The device of claim 1, wherein said revolving means (18) is a handle for manually revolving said clamping means (12).

3. The device of claim 1 or 2, wherein said revolving means (18) is capable of revolving said clamping means (12) in a predetermined angle between over 90° to nearly 180°.

4. The device of any of claims 1 to 3, wherein said data-displaying means (24) is removably connected with a connector for connection and disconnection to a main body (11).

5. The device of claim 4, wherein said connector is capable of being connected with a personal computer.

6. The device of any of claims 1 to 5, wherein said data-displaying means (24) is a printer.

7. The device of any of claims 1 to 6, wherein said sheet material (D) is a corrugated fiberboard.

8. The device of any preceding claim, comprising the features set forth in the ABSTRACT OF THE DISCLOSURE.
